# EUROPÄISCHE PATENTSCHRIFT

(11) **EP 2 670 907 B1**
(45) Veröffentlichungstag und Bekanntmachung des Hinweises auf die Patenterteilung: **20.04.2016**
(21) Anmeldenummer: 12702031.1
(22) Anmeldetag: 01.02.2012
(51) Int. Cl.: A41D 1/00, G01N 33/18, D06M 23/12, D06P 1/00, A61F 13/42, A61L 2/28, G01N 31/22

(54) **KLEIDUNGSSTÜCK**
ARTICLE OF CLOTHING
PIÈCE VESTIMENTAIRE

(30) Priorität: 02.02.2011 DE 102011003517
(43) Veröffentlichungstag der Anmeldung: 11.12.2013
(73) Patentinhaber: Fraunhofer-Gesellschaft zur Förderung der angewandten Forschung e.V., 80686 München (DE)
(72) Erfinder: TRUPP, Sabine, 07318 Saalfeld (DE); STICH, Matthias, 93051 Regensburg (DE); MOHR, Gerhard, 93047 Regensburg (DE)
(74) Vertreter: Schenk, Markus
(86) Internationale Anmeldenummer: PCT/EP2012/051620
(87) Internationale Veröffentlichungsnummer: WO 2012/104328

(56) Entgegenhaltungen:
- EP-A2- 0 403 876
- DE-A1- 2 747 434
- US-A1- 2008 145 945
- US-A1- 2009 064 919

## Beschreibung

Die vorliegende Erfindung bezieht sich auf Kleidungsstücke und andererseits auf die Analyterkennung, wie z.B. die pH-Werterkennung.

Bei sportlichen Aktivitäten oder anderen Anstrengungen kommt man schnell ins Schwitzen. Oft ist den Personen die Tatsache des deutlichen Wasserverlustes durch das Schwitzen nicht gegenwärtig, da das Verdunsten der Schweißes einen Kühleffekt erzielt. Ein zu hoher Wasserverlust kann aber Schwindel, Herzrhythmusstörungen, Nierenversagen oder Bewusstlosigkeit zur Folge haben. Es ist keine Methode bekannt, die eine direkte Information über den aufgetretenen Wasserverlust an die (sportlich) aktive Person gibt.

Weiterhin lassen sich im Schweiß von Personen eine Vielzahl von Analyten nachweisen, die einerseits Aussagen über die Fitness treffen lassen und sogar Hinweise auf vorliegende Erkrankungen liefern können. Bisherige Methoden zum Nachweis von Analyten im Körperschweiß befinden sich im Entwicklungsstadium, erfordern einen hohen apparativen Aufwand und ermöglichen keine direkte Vor-Ort-Diagnostik.

Es wäre wünschenswert, wenn der Nachweis von Analyten im Körperschweiß ohne hohen apparativen Aufwand und in einem möglichst frühen Stadium ihres Auftretens im Körperschweiß stattfinden könnte.

Die im Folgenden beschriebenen Offenlegungsschriften beschreiben ein Färben von Fasern und Faserstücken mit pH-Indikatorfarbstoffen durch Tränken der Fasern mit Farbstofflösungen und eine dadurch erreichte physikalische Haftung der Farben an den Fasern, bzw. Faserstücken. Die Ausführung dieser Techniken ist spielerisch oder modisch orientiert.

In der DE 1478608 wird vor dem Hintergrund einer Spielzeugperücke das Einfärben eines haarartigen Faserbesatzes beschrieben, wobei "der haarartige Faserbesatz mit einem Farbstoff eingefärbt ist, der bei der Änderung des pH-Wertes einen Farbumschlag erfährt und somit einen beliebig wiederholbaren Farbwechsel durch Behandeln des Faserbesatzes mit Flüssigkeiten unterschiedlicher pH-Werte ermöglicht.

Die DE 1603454 nimmt auf die DE 1478608 Bezug und erweitert die dortige Idee, indem generell das Einfärben von Fasern mit pH-Indikatorfarbstoffen beschrieben wird.

Beide Offenlegungsschriften beschreiben das Färben von Fasern mit pH-Indikatorfarbstoffen wie z.B. Methylrot, Alizarin oder Kongorot. Die genannten Methoden erreichen dabei das Färben kompletter Fasern oder auch nur das Färben von Teilbereichen der Faserstücke, bzw. das Drucken von Mustern. Jedoch handelt es sich bei den vorgestellten Fasern um Acetatfasern, Viskosefasern und Baumwollstoffe, welche nach den beschriebenen Verfahren mit den Farbstofflösungen getränkt, getrocknet und gereinigt werden. Aufgrund der Struktur der genannten Fasern wird zwar eine physikalische Haftung der pH-Indikatorfarbstoffe an den Faserzwischenräumen erreicht, aber die Stabilität der Färbung ist so gering, dass mit jedem Waschvorgang durch Verdünnungseffekte ein Austragen der Farben aus den Fasern erfolgen wird.

Für den diagnostischen Einsatz sind die genannten dort beschrieben Techniken nicht geeignet.

Daneben ist in dem Zusammenhang mit dem normalen optischen Färben von Textilien bekannt, dauerhafte chemische Anbindungen von Farbstoffen an Fasern zu verwenden, um einen bleibenden und stabilen Farbeindruck zu erhalten.

In der DE 200 2005 006 986 U1 mit dem Titel "Dauerhaft gefärbte Textilien" wird beispielsweise die chemische Reaktion von Farbstoffen mit Baumwollfasern beschrieben. Ziel ist dabei, eine besonders hohe Farbechtheit beim Färben von Textilien zu erzielen.

Die US 2008/145945 A1 beschreibt eine Lateralfluss-Testvorrichtung zum Testen einer Körperflüssigkeit, wie Urin, Blut, Schleim, Speichel, usw.

Die DE 278 47 434 A1 beschreibt Polymere pH-Farbindikatoren, ihre Herstellung und Verwendung.

Die EP 0 403 876 A2 beschreibt ein Verfahren zur Bestimmung der Gebrauchsintensität von Textilstücken und eine Vorrichtung zur Durchführung des Verfahrens.

Die US 2009/064919 A1 beschreibt ein Verfahren zur Bestimmung, ob ein Kleidungsstück verwendet worden ist. Hierzu wird ein Indikator-Tag an einem Kleidungsstück mit einem Indikatormaterial integriert, das mit einem der Körper Schweiß, Wasser oder Trockenreinigung Chemikalien reagiert.

Es wäre also wünschenswert, wenn es möglich wäre, den Körperschweiß als potenziellen Hinweisgeber für vorliegende Erkrankungen einer Person möglichst frühzeitig, ohne Aufwand für die Person und darüber hinaus im Alltag ausnutzen zu können.

Die Aufgabe der vorliegenden Erfindung besteht dementsprechend darin, ein Konzept hierfür zu schaffen.

Diese Aufgabe wird durch das Kleidungsstück gemäß Anspruch 1 gelöst.

Ein erfindungsgemäßes Kleidungsstück umfasst einen Indikatorfarbstoff, der per chemischer Bindung an das Kleidungsstück angebunden ist.

Die vorliegende Erfindung beruht auf der Erkenntnis, dass eine für die entsprechende Person einfachste Art der Ausnutzung des Hinweischarakters von Körperschweiß für mögliche Erkrankungen darin besteht, ein Kleidungsstück als Träger für einen entsprechenden Indikatorfarbstoff zu verwenden, und zwar ohne zusätzliche Belastungen im Alltag oder zusätzlichen Aufwand allgemeiner Natur, indem der Indikatorfarbstoff per chemischer Bindung, wie z.B. einer Atombindung bzw. kovalenten Bindung, an das Kleidungsstücks angebunden ist. Die chemische Bindung kann unmittelbar zwischen Indikatorfarbstoff und zumindest einer Faser des Kleidungsstücks erfolgen, oder die chemische Bindung kann mittelbar zwischen Indikatorfarbstoff und Kleidungsstück erfolgen, wie z.B. zwischen dem Indikatorfarbstoff und Nano- oder Mikropartikeln vorliegen, die ihrerseits wiederum auf jegliche Art und Weise, wie z.B. chemisch, mechanisch oder über Kleber, mit dem Kleidungsstück verbunden sein können.

Vorteilhafte Ausgestaltungen sind Gegenstand der abhängigen Patentansprüche. Bevorzugte Ausführungsbeispiele werden nachfolgend Bezug nehmend auf die Zeichnungen näher erläutert. Es zeigen:
- Fig. 1: eine schematische Darstellung eines Kleidungsstücks gemäß einem Ausführungsbeispiel; und
- Fig. 2: eine schematische ausschnittweise Schnittansicht eines Kleidungsstücks gemäß einem Ausführungsbeispiel.

Nachfolgend werden Bezug nehmend auf die Zeichnungen Ausführungsbeispiele für Kleidungsstücke beschrieben, bei denen Indikatorfarbstoffe an Fasern bzw. Faserstücke per chemischer Anbindung angebunden sind, wie z.B. mit dem Ziel einer diagnostischen Verwertung der Änderung der optischen Eigenschaften der Indikatorfarbstoffe bei Analyterkennung bzw. Kontaktieren eines entsprechenden Analyts.

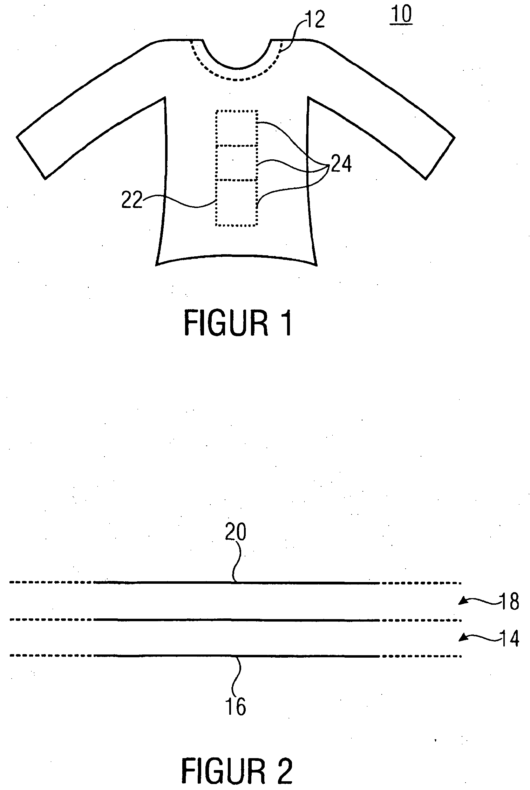

Fig. 1 zeigt beispielsweise ein Kleidungsstück 10. In Fig. 1 ist das Kleidungsstück 10 exemplarisch als ein Oberteil dargestellt, aber es könnte sich ebenso um eine Hose, eine Unterhose, ein Unterhemd, ein T-Shirt, ein Stirnband, ein Schweißband für das Handgelenk, Bademode oder dergleichen handeln.

Das Kleidungsstück 10 umfasst zumindest eine Faser an die per chemischer Bindung ein Indikatorfarbstoff angebunden ist. Beispielsweise besteht das Kleidungsstück 10 zu weniger als 90 Gew.% aus Fasern, an die Indikatorfarbstoff angebunden ist. Das Kleidungsstück 10 kann beispielsweise ausschließlich aus solchen Fasern bestehen, inklusive oder exklusive der Fäden an optionalen Nahtstellen 12 des Kleidungsstücks 10.

Es kann sich allerdings auch genau umgekehrt verhalten, nämlich dass das Kleidungsstück 10 mit dem Indikatorfarbstoff versehene Fasern nur an einer Naht bzw. Nähten 12 aufweist. Das Kleidungsstück 10 kann zudem mehrlagig sein, wie z.B. Innenfutter und Außenfutter aufweisen, wie es in Fig. 2 angedeutet ist, wo das Bezugszeichen 14 einen Stoff anzeigt, der an eine Innenseite 16 des Kleidungsstücks 10 angrenzt, und das Bezugszeichen 18 Stoff anzeigt, der an eine Außenseite 20 des Kleidungsstücks angrenzt. Die Fasern des Textilstoffes 16 sind mit dem Indikatorfarbstoff versehen so dass bei einem Tragen des Kleidungsstücks 10 durch die tragende Person der Indikatorfarbstoff mit der Hautoberfläche der Person in Kontakt steht, so dass die Empfindlichkeit der Erkennung des Analyten in dem Körperschweiß, für den der Indikatorfarbstoff empfindlich ist, erhöht ist. Der Farbausschlag oder die andere optische Eigenschaftsänderung des Indikatorfarbstoffs bei Kontakt mit dem Analyten ist dann von außen für Dritte in diesem Fall nicht sichtbar, was aus ästhetischen oder aber auch aus Datenschutz-Gründen - im Fall des Einsatzes in betreuten Einrichtungen - gewünscht sein kann. Wiewohl die Empfindlichkeit gegenüber dem Analysten insgesamt wohl aufgrund der mittelbaren Kontaktierung der Hauptoberfläche etwas reduziert wäre, bestünde der Vorteil darin, dass der Indikatorfarbstoff des Textilstoffes 20 in diesem Fall nicht direkt auf der Haut aufläge und somit gegebenenfalls weniger Beanspruchung ausgesetzt wäre, durch welche Beanspruchung die Gefahr bestünde, dass Indikatorfarbstoff doch auf die Hautoberfläche der Person gelangt. Wie bereits oben erwähnt, wäre es aber auch möglich, dass ein Textilstoff, der mit dem Indikatorfarbstoff versehen ist, durchgehend von der Innen- zu der Außenseite 20 vorliegt, d. h. das Kleidungsstück 10 mehr oder weniger vollständig aus diesem Stoff besteht.

Es ist aber ferner möglich, dass lediglich Teile des Kleidungsstücks mit einem Fasertextilstoff versehen sind, d.h. nur bestimmte laterale Abschnitte, wie z.B. der Ärmel, eine bestimmte Rückenpartie oder dergleichen, die mit dem Rest des Kleidungsstücks 10 vernäht oder anders verbunden sein können.

Auf die letztgenannte Art und Weise wäre es ebenfalls möglich, dass verschiedene Stellen bzw. Abschnitte des Kleidungsstücks mit Indikatorfarbstoffen versehen sind, die für unterschiedliche Analyten selektiv empfindlich sind, wobei Beispiele für verschiedene Analyten und Indikatorfarbstoffe noch im Folgenden geliefert werden. Durch das örtliche Trennen dieser unterschiedlichen Indikatorfarbstoffe wäre es möglich, eine Überlagerung der verschiedenen optischen Eigenschaftsänderungen der unterschiedlichen Indikatorfarbstoffe im Auge des Betrachters zu vermeiden. Die Prüfung der optischen Eigenschaftsänderungen könnte vielmehr auf unterschiedliche Abschnitte des Kleidungsstücks 10 verteilt werden, wie z.B. auf unterschiedliche Abschnitte, die entlang der Rückenpartie in Wirbelsäulenrichtung der Reihe nach angeordnet sind. Die Abschnitte könnten durch Markierungen von außen optisch sichtbar durch einen Aufdruck oder entsprechende Nähte oder dergleichen voneinander abgegrenzt sein, wobei solche Abgrenzungsmarkierungen in Fig. 1 exemplarisch bei 22 angedeutet sind und die einzelnen Abschnitte mit jeweils einem jeweiligen Indikatorfarbstoff mit dem Bezugszeichen 24.

Mit einem Kleidungsstück gemäß einem der vorhergehenden Ausführungsbeispiele ist es möglich, eine diagnostische Verwertung der Aussagekraft des Körperschweißes einer Person leichter auszunutzen. Die leichte Anwendbarkeit wird unterstützt durch eine stabile chemische Bindung der Indikatorfarbstoffe an die Fasern. Die Fasern erfahren nicht nur eine physikalische Haftung, sondern eben eine chemische Bindung an die Fasern. Die Fasern bzw. Faserstücke können beispielsweise bei 60° bis 90° waschbar sein, ohne dass ein Ausbleichen der Farbe stattfindet. Durch die chemische Bindung der reaktiven Indikatorfarbstoffe an die Fasern sind die Fasern dauerhaft mit dem jeweiligen Indikatorfarbstoff gefärbt. Ausführungsbeispiele der vorliegenden Erfindung sind somit für diagnostische Zwecke geeignet. Die Eignung für diagnostische Zwecke wird dadurch, dass die Fasern bzw. Faserstücke mit Indikatorfarbstoffen chemisch verbunden sind, erleichtert, da eben kein Auswaschen der Indikatorfarbstoffe erfolgen kann.

Wie im Vorhergehenden erwähnt, können Indikatorfarbstoffe eingesetzt werden, die selektiv mit verschiedenen Analyten reagieren. Als Indikatorfarbstoff kann beispielsweise ein pH-Indikatorfarbstoffe verwendet werden, d. h. ein Indikatorfarbstoff, der für die freien H⁺-Ionen oder freien OH⁻-Ionen empfindlich ist. Die Verwendung eines solchen pH-Indikatorfarbstoffes ermöglicht beispielsweise Aussagen über den Wasserverlust der das Kleidungsstück tragenden Person, die beispielsweise eine aktive Person sein kann, wie z.B. ein Sportler. Die sichtbare Farbveränderung kann dabei, wie es im Vorhergehenden ausgeführt ist, vom Anwender selbst oder von anderen Personen ohne apparativen Aufwand erkannt oder gedeutet werden. Der Einsatz anderer Indikatorfarbstoffe zur Erkennung von Analyten, wie z.B. Natriumionen, Glukose, Laktat, Fruktose, reaktiven Sauerstoff-Spezies, Thiolen, Peptiden, Proteinen, Toxinen, Alkoholen oder Aminen kann zur Früherkennung bestimmter Erkrankungen beitragen.

Bei dem Kleidungsstück kann es sich beispielsweise um ein solches handeln, das eine Person regelmäßig in bestimmten Abständen, wie z.B. einmal monatlich über einen bestimmten Zeitraum, trägt, wie z.B. einen Schlafanzug zu bestimmten Nächten. Anhand von eventuell auftretenden Färbungen, die auf das Vorhandensein bestimmter Analyten im Schweiß hinweisen, kann somit eine Früherkennung von Erkrankungen ermöglicht werden und die Person kann sich für weitere Schritte mit einem Arzt in Verbindung setzen.

Es wäre aber ebenfalls die Anwendung eines Kleidungsstücks 10 im Krankenhaus- und Pflegebereich vorteilhaft. Patienten oder zu pflegende Personen könnten durch das Tragen solcher Indikatortextilien zusätzlich hinsichtlich ihres Gesundheitszustandes überwacht werden, und zwar kontinuierlich oder durch intermittierendes Tragen. Auch hier wäre eine Früherkennung bestimmter Erkrankungen oder Veränderungen des Gesundheitszustandes möglich.

Entsprechend den nachzuweisenden Analyten können Indikatorfarbstoffe eingesetzt werden, die Änderungen der optischen Eigenschaften bei Kontakt mit dem jeweiligen Analyten zeigen. Die Analyten können beispielsweise der pH-Wert, Saccharide, Amine, Alkohole, reaktive Sauerstoff-Spezies, Thiole, Peptide, Proteine, Toxine oder Ionen sein.

Die Änderung der optischen Eigenschaften kann sich z.B. durch Änderung der Absorptionswellenlänge, Absorptionsintensität, Emissionswellenlänge, Emissionsintensität oder Fluoreszenzabklingzeit zeigen.

Die chemische Anbindung der reaktiven Indikatorfarbstoffe könnte z.B. durch chemische Reaktion der OH-Gruppen der Zellulosefasern mit Vinylsulfonylgruppen der Indikatorfarbstoffe erfolgen. Dazu werden z.B. 0,5 - 0,02·x Milligramm des reaktiven Indikatorfarbstoffs mit x Mikroliter konzentrierter Schwefelsäure (mit beispielsweise x = 25) versetzt und für 10 bis 60 min, wie z.B. 30 min, bei Raumtemperatur stehengelassen. Dann wird die Mischung in y Milliliter mit z.B. y > x/10, wie z.B. y = 10, destilliertes Wasser gegeben und durch Zugabe von z₁ Mikroliter z₂%-iger Natriumhydroxidlösung neutralisiert (mit z.B. z₁ · z₂ > 100 ·y, wobei z.B. z₁ = 100 und z₂ = 32). Die Zellulose(faser) wird in diese Lösung gegeben. Nach 2-20 min, wie z.B. 5 min, wird eine Lösung von z₃ Gramm Natriumcarbonat in z₄ Milliliter destilliertem Wasser zugegeben, wobei z.B. mit z₃ > x/100 und z₄ > x/16, wie z.B. z₄ = 10 und z₃ = 1,0). Nach weiteren 2-20 min, wie z.B. 5 min, werden 5 · z₁ Mikroliter einer z₂%-igen Natriumhydroxidlösung zugegeben, wobei nach 30 bis 200min, wie z.B. 60 min, die Zellulose(faser) mit destilliertem Wasser gewaschen wird. Durch diese Methode wird eine kovalente Färbung von Fasern, Faserstücken und Textilien mit Indikatorfarbstoffen erreicht.

Gemäß einer anderen Methode könnte die stabile chemische Bindung von Indikatorfarbstoffen an Fasern z.B. durch die Verwendung aminofunktionalisierter Fasern und die chemische Bindung einer Säurefunktion des Indikatorfarbstoffes an die Aminogruppen der Fasern erzielt werden. Die dadurch geknüpfte Amidbindung ist sehr stabil und sorgt ebenfalls für das dauerhafte Färben der Fasern, Faserstücke und Textilien mit Indikatorfarbstoffen.

Ein Vorteil obiger Fasern, Faserstücke bzw. Bekleidungstextilien, die mit chemisch gebundenen Indikatorfarbstoffen ausgestattet sind, ist die einfache Handhabung und der hohe Informationsgehalt der beispielsweise Farbänderung. Eine Farbänderung ist eine leicht zu erkennende und auch leicht zu deutende Information. Dadurch wird es normalen Anwendern ohne hohen apparativen Aufwand und ohne detailliertes Hintergrundwissen möglich, den eigenen Gesundheitszustand bzw. den Gesundheitszustand der zu pflegenden Person z.B. im Krankenhaus oder Pflegebereich zu kontrollieren.

Bezüglich des Ortes der Anwendung der Fasern, Faserstücke und Bekleidungstextilien, die mit chemisch gebundenen Indikatorfarbstoffen ausgestattet sind, besteht ein wichtiger Vorteil in der chemischen Bindung und damit sehr hohen Stabilität der Färbung. Die Fasern, Faserstücke und Bekleidungstextilien können direkt auf der Hautoberfläche der Personen getragen werden, da aufgrund der stabilen Haftung der Fasern ein Austragen der Farbstoffe nicht erfolgt.

Zudem können die Indikatorfarbstoffe reversibel arbeiten. Beispielsweise lassen sie sich durch Waschen in den Ausgangszustand zurückführen. Das Waschen kann dabei bei Temperaturen bis zu 90°C erfolgen, da die Indikatoren chemisch an die Fasern gebunden sind. Die Indikatorfarbstoffe können aber auch irreversibel arbeiten und so eine dauerhafte Farbänderung erzeugen.

Ein weiterer Vorteil ist die Möglichkeit zur Früherkennung von Erkrankungen beizutragen, indem entsprechende Indikatoren an die Fasern gebunden werden.

Entgegen der vorhergehenden Ausführungen ist es gemäß Ausführungsbeispielen der vorliegenden Erfindung so, dass die Indikatorfarbstoffe nicht unmittelbar sondern mittelbar per chemischer Bindung an die Fasern angebunden sind, wie z.B. indem dieselben kovalent an Nano- und Mikropartikel, die beispielsweise auf Cellulose, Dextran, Polyacrylamid, Silika, Gold, Silber oder Quantenpunkten basieren bzw. daraus bestehen, gebunden werden/sind, wobei diese Partikel ihrerseits wiederum kovalent oder nichtkovalent an die Fasern bzw. Textilien gebunden, gedruckt, geklebt oder laminiert werden/sind bzw. anders mit dem Kleidungsstück verbunden sind. Die Nano- und Mikropartikel weisen beispielsweise einen mittleren Durchmesser auf, der zwischen 10 Nanometer und 100 Mikrometer, jeweils einschließlich, liegt. Die Nano-/Mikropartikel können sphärisch geformt sein. Um die Farbstoffe chemisch stabil an Nano- und Mikropartikel zu binden, können sie beispielsweise kovalent an die Oberfläche oder in Poren der Partikel gebunden werden. Alternativ ist es möglich, dass die Nano-/Mikropartikel im Rahmen der Herstellung der Partikel in die Partikel kovalent einpolymerisiert werden. Dadurch können die Partikel in einem separaten Herstellungsprozess mit dem Farbstoff belegt werden, und danach in den üblichen Textildruckverfahren auf die Textilien aufgedruckt werden.

Fasern, Faserstücke und Textilien gemäß obiger Ausführungsbeispiele können somit zur einfachen Diagnostik von Fitness und/oder Gesundheitszustand eingesetzt werden.

Im Vorhergehenden wurde die kovalente Bindung als eine Form der chemischen Bindung beschrieben, die dazu dienen kann, die Indikatorfarbstoffe an die Faser zu binden. Diese chemische Bindung könnte auch als Atombindung beschrieben werden, bei der jeweils ein Atom des Indikatorfarbstoffs und ein Atom der Faser ein Elektron - in gleichen oder zu verschiedenen Anteilen - zur Bindung beiträgt, so dass das Elektronenpaar beiden Atomen angehört.

## Patentansprüche

1. Kleidungsstück, an das per chemischer Bindung ein Indikatorfarbstoff angebunden ist, wobei das Kleindungsstück zumindest eine Faser aufweist, an die der Indikatorfarbstoff mittelbar über Nano- oder Mikropartikel gekoppelt ist, die an die zumindest eine Faser gebunden, gedruckt, geklebt oder laminiert sind, wobei der Indikatorfarbstoff
an die Oberfläche der Nano- oder Mikropartikel kovalent angebunden ist,
kovalent in Poren der Nano- oder Mikropartikel gebunden ist, oder
der Indikatorfarbstoff kovalent in die Nano- oder Mikropartikel einpolymerisiert ist, wobei die zumindest eine Faser an einer Innenseite (16) des Kleidungsstücks freiliegt, so dass bei einem Tragen des Kleidungsstücks durch eine Person der Indikatorfarbstoff mit einer Hautoberfläche der Person in Kontakt steht, so dass eine Empfindlichkeit der Erkennung des Analyten in einem Körperschweiß der Person, für den der Indikatorfarbstoff empfindlich ist, erhöht ist.

2. Kleidungsstück gemäß Anspruch 1, bei dem der Indikatorfarbstoff so ausgebildet ist, dass sich selektiv bei Kontakt mit einem Analyten eine optische Eigenschaft des Indikatorfarbstoffs verändert.

3. Kleidungsstück gemäß Anspruch 2, bei dem der Indikatorfarbstoff so ausgebildet ist, dass die optische Eigenschaft, die sich aufgrund von Kontakt mit dem Analyten selektiv ändert, eine Absorptionswellenlänge, eine Absorptionsintensität, eine Emissionswellenlänge, eine Emissionsintensität oder eine Fluoreszenzabklingzeit des Indikatorfarbstoffs ist.

4. Kleidungsstück gemäß einem der Ansprüche 1 bis 3, bei dem der Indikatorfarbstoff so ausgebildet ist, dass die Veränderung durch Wegwaschen des Analyten reversibel ist.

5. Kleidungsstück gemäß einem der vorhergehenden Ansprüche, bei dem die chemische Bindung eine Atombindung ist.

6. Kleidungsstück gemäß einem der Ansprüche 1 bis 5, bei dem die zumindest eine Faser an einer Außenseite (20) des Kleidungsstücks freiliegt, so dass bei einem Tragen des Kleidungsstücks durch eine Person die Veränderung der optischen Eigenschaft des Indikatorfarbstoffs für Dritte optisch sichtbar ist.

7. Kleidungsstück gemäß einem der vorhergehenden Ansprüche, bei dem der Analyt aus einer Gruppe ausgewählt ist, die aus einem pH-Wert, Sacchariden, Aminen, Natriumionen, Glukose, Laktat, reaktiven Sauerstoff-Spezies, Thiolen, Peptiden, Proteinen, Toxinen, Alkoholen und Fructose besteht.

8. Kleidungsstück gemäß einem der vorhergehenden Ansprüche, bei dem die zumindest eine Faser eine Zellulosefaser ist und die chemische Bindung aus einer chemischen Reaktion von OH-Gruppen der Zellulosefaser mit einer Vinylsulfongruppe des Indikatorfarbstoffs resultiert.

9. Kleidungsstück gemäß einem der vorhergehenden Ansprüche, bei dem die zumindest eine Faser eine aminofunktionalisierte Faser ist und die chemische Bindung eine Amidbindung zwischen einer Säurefunktion des Indikatorfarbstoffs und einer Aminogruppe der aminofunktionalisierten Faser ist.

## Claims

1. A piece of clothing to which an indicator dye is bonded by chemical bonding, wherein the piece of clothing comprises at least one fiber to which the indicator dye is indirectly coupled via nano- or microparticles which are bonded, printed, adhered or laminated to the at least one fiber, wherein the indicator dye is covalently bonded to the surface of the nano- or microparticles,
is covalently bonded in pores of the nano- or microparticles, or
the indicator dye is covalently polymerized into the nano- or microparticles, wherein the at least one fiber is exposed at an interior side (16) of the piece of clothing, so that when the piece of clothing is worn by a person the indicator dye is in contact with a skin surface of the person such that a sensitivity of detection of the analyte in body perspiration of the person for which the indicator dye is sensitive is increased.

2. The piece of clothing according to claim 1, wherein the indicator dye is implemented such that an optical characteristic of the indicator dye selectively changes when contacting an analyte.

3. The piece of clothing according to claim 2, wherein the indicator dye is implemented such that the optical characteristic that changes selectively on contact with the analyte, is an absorption wavelength, an absorption intensity, an emission wavelength, an emission intensity or a fluorescence decay time of the indicator dye.

4. The piece of clothing according to one of claims 1 to 3, wherein the indicator dye is implemented such that the change is reversible by washing out the analyte.

5. The piece of clothing according to one of the preceding claims, wherein the chemical bond is an atomic bond.

6. The piece of clothing according to one of claims 1 to 5, wherein the at least one fiber is exposed at an exterior side (20) of the piece of clothing, so that when the piece of clothing is worn by a person the change in the optical characteristic of the indicator dye is optically visible to a third person.

7. The piece of clothing according to one of the preceding claims, wherein the analyte is selected from a group consisting of a pH value, saccharides, amines, sodium ions, glucose, lactate, reactive oxygen species, thiols, peptides, proteins, toxins, alcohols and fructose.

8. The piece of clothing according to one of the preceding claims, wherein the at least one fiber is a cellulose fiber and the chemical bond results from a chemical reaction of OH groups of the cellulose fiber with a vinylsulfone group of the indicator dye.

9. The piece of clothing according to one of the preceding claims, wherein the at least one fiber is an amino functionalized fiber and the chemical bond is an amide bond between an acid function of the indicator dye and an amino group of the amino functionalized fiber.

## Revendications

1. Vêtement auquel est lié, par liaison chimique, un colorant indicateur, le vêtement présentant au moins une fibre à laquelle le colorant indicateur est couplé indirectement par l'intermédiaire de nanoparticules ou de microparticules qui sont liées à, imprimées, collées ou stratifiées sur l'au moins une fibre, le colorant indicateur
étant lié de manière covalente à la surface des nanoparticules ou microparticules,
étant lié de manière covalente dans les pores des nanoparticules ou microparticules, ou
le colorant indicateur étant polymérisé de manière covalente dans les nanoparticules ou microparticules,
dans lequel l'au moins une fibre d'un côté intérieur (16) du vêtement est exposée, de sorte que, lorsque le vêtement est porté par une personne, le colorant indicateur est en contact avec une surface de la peau de la personne, de sorte qu'une sensibilité de la détection de l'analyte dans un sueur corporelle de la personne auquel le colorant indicateur est sensible soit augmentée.

2. Vêtement selon la revendication 1, dans lequel le colorant indicateur est réalisé de sorte qu'au contact avec un analyte varie sélectivement une propriété optique du colorant indicateur.

3. Vêtement selon la revendication 2, dans lequel le colorant indicateur est réalisé de sorte que la caractéristique optique qui varie sélectivement du fait du contact avec l'analyte est une longueur d'onde d'absorption, une intensité d'absorption, une longueur d'onde d'émission, une intensité d'émission ou un temps d'affaiblissement de fluorescence du colorant indicateur.

4. Vêtement selon l'une des revendications 1 à 3, dans lequel le colorant indicateur est réalisé de sorte que la variation soit réversible par élimination par lavage de l'analyte.

5. Vêtement selon l'une quelconque des revendications précédentes, dans lequel la liaison chimique est une liaison atomique.

6. Vêtement selon l'une des revendications 1 à 5, dans lequel l'au moins une fibre est exposée d'un côté extérieur (20) du vêtement, de sorte que, lorsque le vêtement est porté par une personne, la variation de la propriété optique du colorant indicateur est visible optiquement pour des tiers.

7. Vêtement selon l'une des revendications précédentes, dans lequel l'analyte est choisi parmi un groupe composé d'une valeur de pH, de saccharides, d'amines, d'ions de sodium, de glucose, de lactate, d'espèces d'oxygène réactif, de thiols, de peptides, de protéines, de toxines, d'alcools et de fructose.

8. Vêtement selon l'une des revendications précédentes, dans lequel l'au moins une fibre est une fibre de cellulose et la liaison chimique résulte d'une réaction chimique de groupes OH de la fibre de cellulose avec une groupe vinylsulfone de l'indicateur de colorant.

9. Vêtement selon l'une des revendications précédentes, dans lequel l'au moins une fibre est une fibre fonctionnalisée par un groupe amino et la liaison chimique est une liaison amide entre une fonction acide du colorant indicateur et un groupe amino de la fibre fonctionnalisée par un groupe amino.
